(19) Europäisches Patentamt / European Patent Office / Office européen des brevets

(11) **EP 4 579 684 A1**

(12) **EUROPEAN PATENT APPLICATION**
published in accordance with Art. 153(4) EPC

(43) Date of publication:
**02.07.2025  Bulletin 2025/27**

(21) Application number: **23856878.6**

(22) Date of filing: **23.03.2023**

(51) International Patent Classification (IPC):
**G16H 50/50** (2018.01)

(52) Cooperative Patent Classification (CPC):
**G16H 50/50**

(86) International application number:
**PCT/JP2023/011378**

(87) International publication number:
**WO 2024/042757 (29.02.2024 Gazette 2024/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **22.08.2022  JP 2022132055**

(71) Applicant: **Tohoku University**
**Sendai-shi, Miyagi 980-8577 (JP)**

(72) Inventor: **YAMADA Yosuke**
**Sendai-shi, Miyagi 980-8577 (JP)**

(74) Representative: **V.O.**
**P.O. Box 87930**
**2508 DH Den Haag (NL)**

(54) **MODEL DETERMINATION SYSTEM, WATER METABOLISM INDEX ESTIMATION SYSTEM, HEALTH DEGREE ESTIMATION SYSTEM, MODEL DETERMINATION METHOD, WATER METABOLISM INDEX ESTIMATION METHOD, HEALTH DEGREE ESTIMATION METHOD, AND PROGRAM**

(57)     A water metabolism index estimation device (1) includes a physical information acquisition unit (13) and a water metabolism index estimation unit (14). The physical information acquisition unit (13) is configured to acquire second physical information numerically indicating a physical feature of a subject to be estimated. The water metabolism index estimation unit (14) is configured to acquire an index model for deriving, from physical information indicating a physical feature, the water metabolism index indicating the degree of water metabolism, the index model being determined by, for a plurality of subjects to be stored, associating one or a plurality of pieces of first physical information numerically indicating a physical feature of the subject to be stored with a first water metabolism index indicating the degree of water metabolism of the subject to be stored in a predetermined period, and determine, by applying second physical information to the index model, a second water metabolism index indicating the degree of water metabolism of the subject to be estimated.

FIG.1

EP 4 579 684 A1

**Description**

Technical Field

The present disclosure relates to a model determination device, a water metabolism index estimation device, a health degree estimation device, a model determination method, a water metabolism index estimation method, a health degree estimation method, and a program.

Background Art

As an example of health management, a technique has been developed to prevent subjects of health management from becoming dehydrated. As an example of this type of technique, Patent Literature 1 discloses a dehydration state management system that estimates the dehydration state of a subject. The dehydration state management system disclosed in Patent Literature 1 estimates the dehydration state of the subject based on blood flow data, the amount of water supplied by a water supply device, and the environmental state.

Citation List

Patent Literature

Patent Literature 1: JP2022-42721A

Summary of Invention

Technical Problem

In order to prevent the dehydration state, it is necessary to prompt a subject of health management to take an appropriate amount of water. The dehydration state management system disclosed in Patent Literature 1 can estimate the dehydration state of the subject, but cannot indicate the amount of water required by the subject to prevent the dehydration state.

The amount of water to be taken can be determined based on an index indicating the degree of water metabolism, specifically, the water balance which is the amount of water exchanged in the body each day. There is a doubly-labelled water method as a method for determining the water balance, in other words, the water turnover. In the doubly-labelled water method, water containing a stable isotope $^2$H of hydrogen and a stable isotope $^{18}$O of oxygen is administered to a subject to be measured, and total body water, the total energy expenditure, the water balance, and the like, are determined based on excretion rates of the two stable isotopes obtained by analyzing attenuation rates of the stable isotopes in the body fluid of the subject to be measured over a measurement period of, for example, two weeks.

The water balance can be determined by using the doubly-labelled water method, but in order to perform the doubly-labelled water method, a device for analyzing water containing a stable isotope and the stable isotope is required, and further, as described above, it is necessary to measure the body fluid of the subject to be measured over the measurement period, so that special equipment and time are required. In other words, since estimation processing of the water balance using the doubly-labelled water method is complicated and takes time, it is difficult to routinely determine the water balance based on the doubly-labelled water method for each of a large number of subjects of health management.

The present disclosure has been made in view of the above-described circumstances, and an object of the present disclosure is to provide a model determination device, a water metabolism index estimation device, a health degree estimation device, a model determination method, a water metabolism index estimation method, a health degree estimation method, and a program capable of simply determining the water metabolism index.

Solution to Problem

In order to achieve the above object, a model determination device according to a first aspect of the present disclosure includes:

a storage unit configured to store, for a plurality of subjects to be stored, one or a plurality of pieces of first physical information numerically indicating a physical feature of the subject to be stored and a first water metabolism index indicating the degree of water metabolism of the subject to be stored in a predetermined period in association with each other; and
a determination unit configured to determine, based on the association of the first physical information and the first water metabolism index of each of the subjects to be stored, an index model for deriving, from physical information indicating a physical feature, the water metabolism index indicating the degree of water metabolism.

Preferably, the determination unit determines, by regression analysis using the first physical information as an explanatory variable and the first water metabolism index as an objective function, one or a plurality of first parameters for weighting each piece of the physical information to obtain the water metabolism index.

Preferably, the determination unit learns the first physical information and the first water metabolism index, and determines the index model, which is a neural network model that receives the physical information as an input, and outputs the water metabolism index.

Preferably, the storage unit stores, for the plurality of subjects to be stored, the first physical information, the first water metabolism index, and one or a plurality of pieces of first environmental information numerically indicating the environmental state around the subject to be stored, in association with each other.

Preferably, the determination unit determines, based on the association of the first physical information, the first water metabolism index, and the first environmental information of each of the subjects to be stored, the index model for deriving the water metabolism index from the physical information and environmental information numerically indicating an environmental state.

Preferably, the determination unit determines, by regression analysis using the first physical information and the first environmental information as explanatory variables and using the first water metabolism index as an objective function, a plurality of first parameters for weighting each of the physical information and the environmental information to obtain the water metabolism index.

Preferably, the determination unit determines the plurality of first parameters by multiple regression analysis using, as explanatory variables, the first physical information including the physical activity level, the weight, the gender, the athlete level, and the age, and the first environmental information including the temperature, the humidity, and the human development index, and using the first water metabolism index as an objective function.

Preferably, the determination unit learns the first physical information, the first water metabolism index, and the first environmental information, and determines the index model, which is a neural network model that receives the physical information and the environmental information as inputs, and outputs the water metabolism index.

Preferably, the storage unit stores the first water metabolism index determined based on a doubly-labelled water method.

A water metabolism index estimation device according to a second aspect of the present disclosure includes:

a physical information acquisition unit configured to acquire one or a plurality of pieces of second physical information numerically indicating a physical feature of a subject to be estimated; and

a water metabolism index estimation unit configured to acquire an index model for deriving, from physical information indicating a physical feature, a water metabolism index indicating the degree of water metabolism, the index model being determined by associating, for a plurality of subjects to be stored, one or a plurality of pieces of first physical information numerically indicating a physical feature of the subject to be stored with a first water metabolism index indicating the degree of water metabolism of the subject to be stored in a predetermined period, and determine, by applying the second physical information to the index model, a second water metabolism index indicating the degree of water metabolism of the subject to be estimated.

Preferably, the physical information acquisition unit acquires one or a plurality of pieces of the second physical information which are measurement values of the physical feature of the subject to be estimated, and one or a plurality of other pieces of the second physical information estimated from the second physical information.

Preferably, the physical information acquisition unit acquires one or a plurality of pieces of the second physical information which are measurement values of the physical feature of the subject to be estimated, and one or a plurality of other pieces of the second physical information whose values are determined in advance.

Preferably, the physical feature includes at least one of the gender, the age, the weight, the fat free mass, the physical activity level, the total energy expenditure, the socioeconomic status, and the athlete level indicating the daily exercise intensity.

Preferably, the water metabolism index estimation device further includes an environmental information acquisition unit configured to acquire one or a plurality of pieces of second environmental information numerically indicating the environmental state around the subject to be estimated, and

the water metabolism index estimation unit acquires the index model for deriving the water metabolism index from the physical information and environmental information indicating the environmental state, the index model being determined by associating, for the plurality of subjects to be stored, the first physical information, the first water metabolism index, and one or a plurality of pieces of first environmental information numerically indicating the environmental state around the subject to be stored, and determines the second water metabolism index by applying the second physical information and the second environmental information to the index model.

Preferably, the environmental information acquisition unit acquires one or a plurality of pieces of the second environmental information which are measurement values of the environmental state around the subject to be estimated, and one or a plurality of other pieces of the second environmental information estimated from the second environmental information.

Preferably, the environmental information acquisition unit acquires one or a plurality of pieces of the second environmental information which are measurement values of the environmental state around the subject to be estimated, and one or a plurality of other pieces of the second environmental information whose values are determined in advance.

Preferably, the environmental state includes at least one of the temperature, the humidity, the latitude, the altitude, and the human development index.

A health degree estimation device according to a third aspect of the present disclosure includes:

the water metabolism index estimation device described above; and
a health degree estimation unit configured to determine the metabolic health degree by weighting each of the second water metabolism index and the second physical information with a predetermined second parameter.

A model determination method according to a fourth aspect of the present disclosure includes:
determining an index model for deriving, from physical information indicating a physical feature, the water metabolism index indicating the degree of water metabolism by associating, for a plurality of subjects to be stored, one or a plurality of pieces of first physical information numerically indicating a physical feature of the subject to be stored with a first water metabolism index indicating the degree of water metabolism of the subject to be stored in a predetermined period.

A water metabolism index estimation method according to a fifth aspect of the present disclosure includes:

acquiring one or a plurality of pieces of second physical information numerically indicating a physical feature of a subject to be estimated; and
acquiring an index model for deriving, from physical information indicating a physical feature, a water metabolism index indicating a degree of water metabolism, the index model being determined by associating, for a plurality of subjects to be stored, one or a plurality of pieces of first physical information numerically indicating a physical feature of the subject to be stored with a first water metabolism index indicating a degree of water metabolism of the subject to be stored in a predetermined period, and determining, by applying the second physical information to the index model, a second water metabolism index indicating a degree of water metabolism of the subject to be estimated.

A health degree estimation method according to a sixth aspect of the present disclosure includes:

acquiring one or a plurality of pieces of second physical information numerically indicating a physical feature of a subject to be estimated;
acquiring an index model for deriving, from physical information indicating a physical feature, a water metabolism index indicating a degree of water metabolism, the index model being determined by associating, for a plurality of subjects to be stored, one or a plurality of pieces of first physical information numerically indicating a physical feature of the subject to be stored with a first water metabolism index indicating a degree of water metabolism of the subject to be stored in a predetermined period, and determining, by applying the second physical information to the index model, a second water metabolism index indicating a degree of water metabolism of the subject to be estimated; and
determining a metabolic health degree by weighting each of the second water metabolism index and the second physical information with a predetermined second parameter.

A program according to a seventh aspect of the present disclosure causes a computer to function as:

a storage unit configured to store, for a plurality of subjects to be stored, one or a plurality of pieces of first physical information numerically indicating a physical feature of the subject to be stored and a first water metabolism index indicating the degree of water metabolism of the subject to be stored in a predetermined period in association with each other; and
a determination unit configured to determine, based on the association of the first physical information and the first water metabolism index of each of the subjects to be stored, an index model for deriving, from physical information indicating a physical feature, the water metabolism index indicating the degree of water metabolism.

A program according to an eighth aspect of the present disclosure causes a computer to function as:

a physical information acquisition unit configured to acquire one or a plurality of pieces of second physical information numerically indicating a physical feature of a subject to be estimated; and

a water metabolism index estimation unit configured to acquire an index model for deriving, from physical information indicating a physical feature, the water metabolism index indicating the degree of water metabolism, the index model being determined by associating, for a plurality of subjects to be stored, one or a plurality of pieces of first physical information numerically indicating a physical feature of the subject to be stored with a first water metabolism index indicating the degree of water metabolism of the subject to be stored in a predetermined period, and determine, by applying the second physical information to the index model, a second water metabolism index indicating a degree of water metabolism of the subject to be estimated.

A program according to a ninth aspect of the present disclosure causes a computer to function as:

a physical information acquisition unit configured to acquire one or a plurality of pieces of second physical information numerically indicating a physical feature of a subject to be estimated;

a water metabolism index estimation unit configured to acquire an index model for deriving, from physical information indicating a physical feature, a water metabolism index indicating the degree of water metabolism, the index model being determined by associating, for a plurality of subjects to be stored, one or a plurality of pieces of first physical information numerically indicating a physical feature of the subject to be stored with a first water metabolism index indicating the degree of water metabolism of the subject to be stored in a predetermined period, and determine, by applying the second physical information to the index model, a second water metabolism index indicating the degree of water metabolism of the subject to be estimated; and

a health degree estimation unit configured to determine the metabolic health degree by weighting each of the second water metabolism index and the second physical information with a predetermined second parameter.

Advantageous Effects of Invention

According to the present disclosure, a water metabolism index can be simply determined based on an index model for deriving the water metabolism index from physical information.

Brief Description of Drawings

FIG. 1 is a block diagram of a model determination device, a water metabolism index estimation device, and a health degree estimation device according to Embodiment 1.

FIG. 2 is a diagram illustrating an example of a water metabolism index and first physical information that are stored in a storage unit provided in the model determination device according to Embodiment 1.

FIG. 3 is a diagram illustrating an example of the correlation between the physical activity level and the water balance in Embodiment 1.

FIG. 4 is a diagram illustrating an example of the correlation between the fat free mass and the water balance in Embodiment 1.

FIG. 5 is a diagram illustrating an example of the correlation between the athlete level and the water balance in Embodiment 1.

FIG. 6 is a diagram illustrating a hardware configuration of the model determination device, the water metabolism index estimation device, and the health degree estimation device according to Embodiment 1.

FIG. 7 is a flowchart illustrating an example of water metabolism index estimation processing performed by the water metabolism index estimation device according to Embodiment 1.

FIG. 8 is a flowchart illustrating an example of health degree estimation processing performed by the health degree estimation device according to Embodiment 1.

FIG. 9 is a block diagram of a model determination device, a water metabolism index estimation device, and a health degree estimation device according to Embodiment 2.

FIG. 10 is a diagram illustrating an example of a water metabolism index, a first physical information, and a first environmental information that are stored in a storage unit provided in the model determination device according to Embodiment 2.

FIG. 11 is a diagram illustrating an example of the relationship between the temperature and the water balance in Embodiment 2.

FIG. 12 is a diagram illustrating an example of the relationship between the human development index and the water balance in Embodiment 2.

FIG. 13 is a flowchart illustrating an example of water metabolism index estimation processing performed by the water metabolism index estimation device according to Embodiment 2.

FIG. 14 is a block diagram of a modification of the model determination device, the water metabolism index estimation device, and the health degree estimation device according to the embodiment.

FIG. 15 is a diagram illustrating a modification of the hardware configuration of the model determination device, the water metabolism index estimation device, and the health degree estimation device according to the embodiment.

Description of Embodiments

Hereinafter, a model determination device, a water metabolism index estimation device, a health degree estimation device, a model determination method, a water metabolism index estimation method, a health degree estimation method, and a program according to embodiments of the present disclosure will be described in detail with reference to the drawings. In the drawings, the same or equivalent parts are denoted by the same reference numerals.

(Embodiment 1)

A model determination device 10 that determines an index model for deriving the water metabolism index from physical information, a water metabolism index estimation device 1 that estimates the water metabolism index indicating the degree of water metabolism of a subject to be estimated who is a subject of health management, and a health degree estimation device 2 that estimates, based on the estimated water metabolism index, the health degree of the subject to be estimated will be described in Embodiment 1. The physical information is information indicating a physical feature, and the water metabolism index is information indicating the degree of water metabolism.

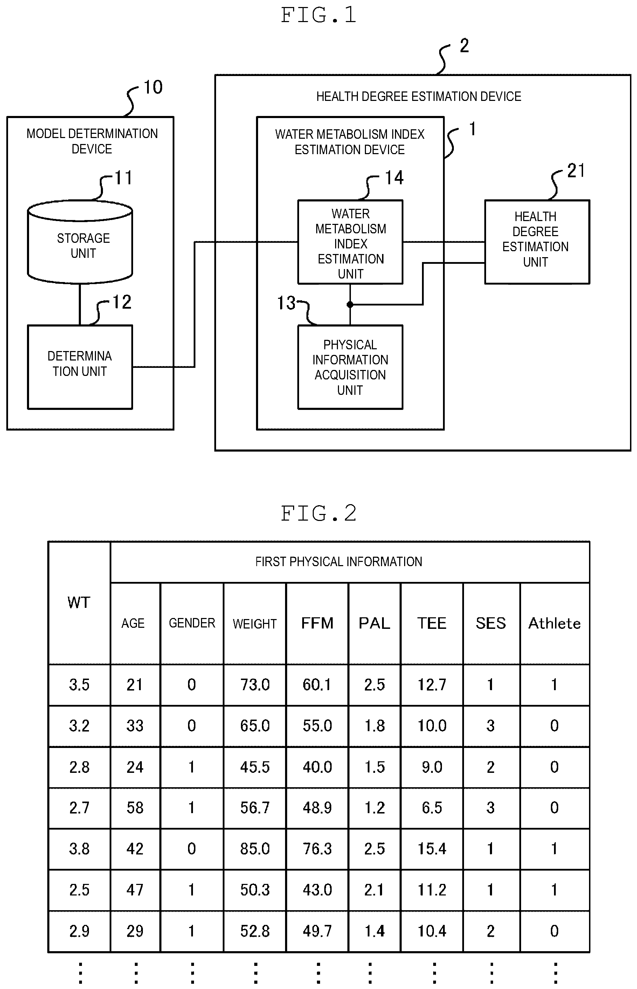

The model determination device 10 illustrated in FIG. 1 includes a storage unit 11 that stores, for a plurality of subjects to be stored, who are a plurality of persons to be analyzed for water metabolism, specifically, to be analyzed based on a doubly-labelled water (DLW) method, one or a plurality of pieces of first physical information and a first water metabolism index of each subject to be stored in association with each other. The first physical information numerically indicates a physical feature of the subject to be stored. The first water metabolism index is an index indicating the degree of water metabolism of each subject to be stored in a predetermined period.

The model determination device 10 further includes a determination unit 12 that determines the index model for deriving the water metabolism index from physical information. The determination unit 12 determines by, for example, regression analysis, one or a plurality of first parameters for weighting each piece of physical information to obtain the water metabolism index.

The water metabolism index estimation device 1 determines a second water metabolism index indicating the degree of water metabolism of a subject to be estimated who is an individual different from the subject to be stored that is to be analyzed for water metabolism. Specifically, the water metabolism index estimation device 1 determines the second water metabolism index by applying one or a plurality of pieces of second physical information numerically indicating a physical feature of the subject to be estimated to the index model determined by the model determination device 10 described above. The water metabolism index estimation device 1 includes a physical information acquisition unit 13 that acquires one or the plurality of pieces of second physical information, and a water metabolism index estimation unit 14 that determines the second water metabolism index by applying the second physical information to the index model. The water metabolism index estimation unit 14 determines the second water metabolism index by weighting each piece of the second physical information with the first parameter determined by the determination unit 12 of the model determination device 10, for example.

The health degree estimation device 2 includes the water metabolism index estimation device 1 including the above-described configuration, and a health degree estimation unit 21 that determines the metabolic health degree of the subject to be estimated by weighting each of the second physical information and the second water metabolism index determined by the water metabolism index estimation unit 14 of the water metabolism index estimation device 1 with a second parameter.

Details of each unit of the model determination device 10 will be described below. The storage unit 11 stores, as the first water metabolism index, the water balance which is the amount of water exchanged in the body each day, in other words, the water turnover (unit: L/day or mL/day). The physical feature includes at least one of the age, the gender, the weight, the fat free mass (FFM), the physical activity level (PAL), the total energy expenditure (TEE), the socioeconomic status (SES), and the athlete level indicating a daily exercise intensity.

In Embodiment 1, as illustrated in FIG. 2, the storage unit 11 stores the water balance WT of each subject to be stored and the age, gender, weight, fat free mass, physical activity level, total energy expenditure, socioeconomic status, and athlete level constituting the first physical information in association with each other. In FIG. 2, the athlete level is illustrated as Athlete. Each record in the table illustrated in FIG. 2 is data corresponding to each subject to be stored.

The water balance WT is the value determined based on the doubly-labelled water method. The table illustrated in FIG. 2 may store values determined based on the doubly-labelled water method and may be composed of data acquired from a publicly available DLW database.

In the doubly-labelled water method, water containing a stable isotope $^2$H of hydrogen and a stable isotope $^{18}$O of oxygen is administered to a subject to be measured. The excretion rate $k_D$ of the stable isotope $^2$H of hydrogen, the excretion rate $k_O$ of the stable isotope $^{18}$O of oxygen, the diluted volume $N_D$ of the stable isotope $^2$H of hydrogen, and the diluted volume $N_O$ of the stable isotope $^{18}$O of oxygen are obtained by analyzing attenuation rates of the stable isotopes in the body fluid of the subject to be measured over a predetermined period, for example, a measurement period of two weeks.

rH$_2$O represented by the following formula (1) is used as the water balance WT. N in the following formula (1) is the average of diluted volumes and is represented by the following formula (2).

$$rH_2O = 1.04\ k_DN \qquad (1)$$

$$N = (N_D/1.043 + N_O/1.007)/2 \qquad (2)$$

As described above, a part of the first physical information associated with the water balance WT determined based on the doubly-labelled water method is derived from data determined by the doubly-labelled water method. Specifically, total body water (TBW) is obtained based on the diluted volume $N_D$ of the stable isotope $^2$H of hydrogen and the diluted volume $N_O$ of the stable isotope $^{18}$O of oxygen, and the fat free mass is obtained based on the total body water by assuming that the hydration rate in the body of an adult is 73.2%.

The excretion rate of carbon dioxide is determined based on the total body water, the excretion rate $k_D$ of the stable isotope $^2$H of hydrogen, and the excretion rate $k_O$ of the stable isotope $^{18}$O of oxygen. The total energy expenditure is determined based on the excretion rate of carbon dioxide using the de Weir indirect calorimetry formula. The physical activity level is determined by dividing the total energy expenditure by the basal energy expenditure (BEE). The basal energy expenditure can be determined based on the height, the weight, and the age using, for example, the Harris-Benedict formula.

The DLW database stores information on physical feature of the subject to be measured, specifically, gender, age, weight, socioeconomic status, and athlete level. The storage unit 11 stores the first physical information indicating these physical features in association with the water balance WT. The gender is assumed to take the value of either 0 indicating a male or 1 indicating a female. The socioeconomic status is assumed to take one of three values: 1 indicating a high standard of living, 2 indicating an average standard of living, and 3 indicating a low standard of living. The athlete level is assumed to take the value of either 1 indicating that the person is an athlete, specifically, a person with a high daily exercise load, or 0 indicating that the person is not an athlete.

The determination unit 12 illustrated in FIG. 1 acquires, as the first water metabolism index, the water balance WT of each subject to be stored that is stored in the storage unit 11 as illustrated in FIG. 2, and acquires the first physical information of each subject to be stored that is stored in the storage unit 11. The determination unit 12 determines a plurality of first parameters for weighting each piece of physical information to obtain the water metabolism index, and sends the first parameters to the water metabolism index estimation device 1. Specifically, the determination unit 12 determines the first parameters by regression analysis using each piece of the first physical information as an explanatory variable and using the first water metabolism index as an objective variable. The determination unit 12 sends the first parameters to the water metabolism index estimation unit 14.

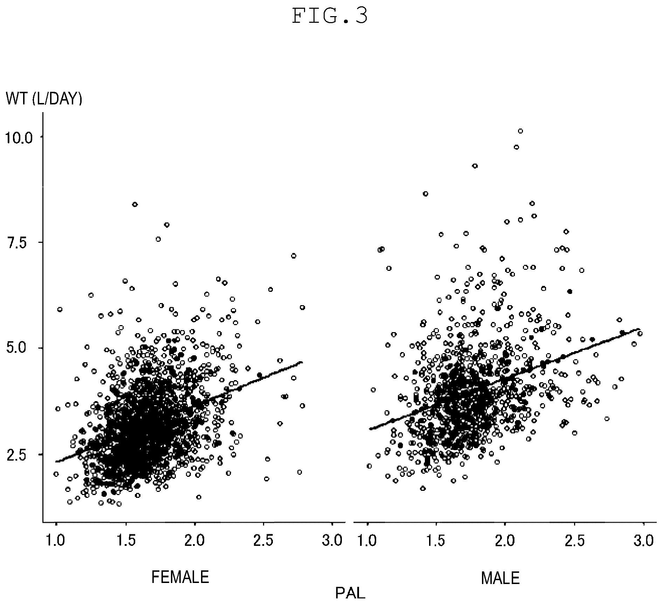

It is preferable that the determination unit 12 determines the first parameter by performing multiple regression analysis using, as an explanatory variable, the first physical information having a high correlation with the first water metabolism index among the first physical information. FIG. 3 illustrates an example of the correlation between the physical activity level PAL, which is an example of the first physical information, and the water balance WT (unit: L/day). The left side of the drawing illustrates the relationship between the physical activity level PAL and the water balance WT of a female, and the right side of the drawing illustrates the relationship between the physical activity level PAL and the water balance WT of a male. The horizontal axis represents the physical activity level PAL, and the vertical axis represents the water balance WT. Since in the analysis performed to determine the correlation degree in FIG. 3, the p-value is less than 0.001, it can be seen that there is a high correlation between the physical activity level PAL and the water balance WT.

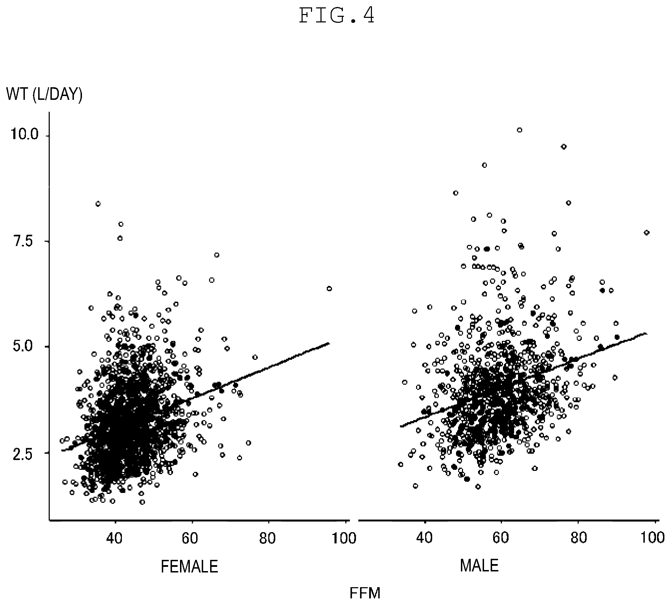

FIG. 4 illustrates an example of the correlation between the fat free mass FFM (unit: kg), which is an example of the first physical information, and the water balance WT (unit: L/day). The left side of the drawing illustrates the relationship between the fat free mass FFM and the water balance WT of a female, and the right side of the drawing illustrates the relationship between the fat free mass FFM and the water balance WT of a male. The horizontal axis represents the fat free mass FFM, and the vertical axis represents the water balance WT. Since in the analysis performed to determine the correlation degree in FIG. 4, the p-value is less than 0.001, it can be seen that there is a high correlation between the fat free mass FFM and the water balance WT.

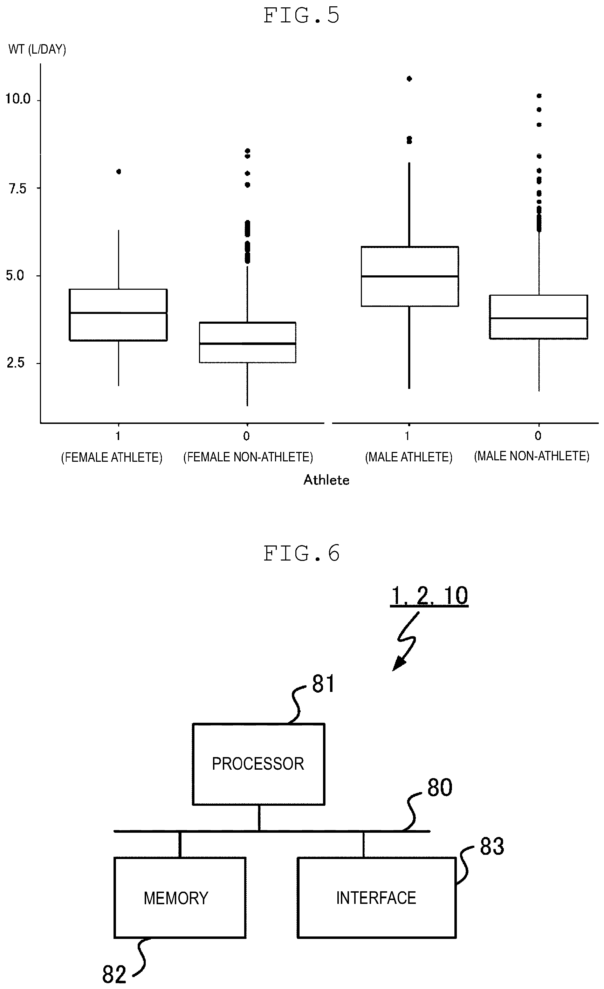

FIG. 5 illustrates an example of the relationship between the athlete level, which is an example of the first physical information, and the water balance WT (unit: L/day). The vertical axis represents the water balance WT. FIG. 5 is a box plot illustrating variations in the water balance WT according to the athlete level and the gender. Graphs illustrating the water balance WT of each of the subjects to be stored that correspond to a female athlete, a female non-athlete, a male athlete, and a male non-athlete are displayed in this order from the left of the drawing. The non-athlete refers to a person who is not an athlete, that is, a person with a low daily exercise load. As illustrated in FIG. 5, in both cases of female and male, the box corresponding to the water balance WT of an athlete is located higher than the box corresponding to the water balance WT of a non-athlete. That is, the athlete level has a positive correlation with the water balance WT.

In order to accurately determine the water balance WT, it is preferable to perform the multiple regression analysis using the first physical information having a high correlation with the water balance WT, such as the physical activity level, the fat free mass, and the athlete level described above. For example, the determination unit 12 determines, by the multiple regression analysis using the first physical information including the gender, the age, the weight, the physical activity level, and the athlete level as an explanatory variable and the water balance WT as an objective variable, the first parameter for weighting the physical information to obtain the water balance.

The model determination device 10 illustrated in FIG. 1 is implemented by data acquired from the DLW database as described above, determines, based on the water balance WT and the first physical information that are stored in the storage unit 11, the first parameter for weighting the physical information to determine the water metabolism index, and sends the first parameter as the index model to the water metabolism index estimation device 1.

Details of the water metabolism index estimation device 1 that acquires the first parameter as the index model from the model determination device 10 described above and determines the water metabolism index of a subject to be estimated will be described below. The subject to be estimated is an individual different from the subject to be stored that is associated with the data stored in the storage unit 11 of the model determination device 10, and is an individual who is a subject of water metabolism index estimation processing.

The physical information acquisition unit 13 provided in the water metabolism index estimation device 1 acquires a physical feature of the subject to be estimated. Specifically, the physical information acquisition unit 13 acquires one or a plurality of pieces of second physical information numerically indicating the physical feature of the subject to be estimated, and sends the acquired second physical information to the water metabolism index estimation unit 14 and the health degree estimation unit 21. The physical feature acquired by the physical information acquisition unit 13 includes the physical feature indicated by the first physical information stored in the storage unit 11. The item of the second physical information is the same as the item of the first physical information, but the first physical information indicates the physical feature of the subject to be stored who is a subject to be analyzed for water analysis, whereas the second physical information indicates the physical feature of the subject to be estimated.

The physical information acquisition unit 13 receives an input of information on the socioeconomic status of the subject to be estimated via an input and output device. The physical information acquisition unit 13 acquires the second physical information, which is the measurement value of the physical feature of the subject to be estimated, and other pieces of second physical information estimated from the second physical information. For example, the physical information acquisition unit 13 acquires, from the body composition meter, the age and the gender of the subject to be estimated that are input to the body composition meter, and the weight and the body fat percentage of the subject to be estimated that are measured by the body composition meter. The physical information acquisition unit 13 determines the fat free mass based on the weight and the body fat percentage. The physical information acquisition unit 13 acquires the heart rate of the subject to be estimated from a heart rate meter, estimates the exercise load based on the acquired heart rate, and estimates the physical activity level and the athlete level. The physical information acquisition unit 13 determines the basal energy expenditure based on the height, the weight, and the age using the Harris-Benedict formula, and determines the total energy expenditure by multiplying the basal energy expenditure by the estimated physical activity level.

The water metabolism index estimation unit 14 weights each piece of the second physical information acquired from the physical information acquisition unit 13 with the first parameter acquired from the determination unit 12 of the model determination device 10 to determine the water balance WT as the second water metabolism index indicating the degree of water metabolism of the subject to be estimated. For example, the water metabolism index estimation unit 14 weights the first physical information including the gender, the age, the weight, the physical activity level, and the athlete level with the first parameter based on the following formula (3) to determine the water balance WT (unit: mL/day). Weighting coefficients a1, a2, a3, a4, and a5 in the following formula (3) are the first parameter. For example, the weighting coefficients a1, a2, a3, a4, and a5 are positive numbers. In the following formula (3), age is represented by age.

$$WT = a1*PAL + a2*weight + a3*gender + a4*Athlete - a5*age \quad (3)$$

The water metabolism index estimation unit 14 sends the obtained second water metabolism index to the health degree estimation unit 21. The water metabolism index estimation unit 14 may send the second water metabolism index to an output device (not illustrated), and output the second water metabolism index by display, sound output, or the like, on the output device to prompt the subject to be estimated to take an appropriate amount of water.

The health degree estimation unit 21 determines the metabolic health degree based on at least a part of the second water metabolism index acquired from the water metabolism index estimation unit 14 and the second physical information acquired from the physical information acquisition unit 13. The metabolic health degree is an index based on a factor that determines the total energy expenditure, and indicates whether metabolism is sufficiently performed. The factor that determines the total energy expenditure is, for example, the physical activity level, the fat free mass, the athlete level, the body fat percentage, and the age. The metabolic health degree is represented by, for example, the following formula (4). Weighting coefficients b1, b2, b3, b4, and b5 in the following formula (4) are positive coefficients. In the following formula (4), the body fat percentage is represented by BFP.

$$\text{Metabolic health degree} = b1*PAL + b2*FFM + b3*Athlete - b4*BFP - b5*age$$

$$(4)$$

The health degree estimation unit 21 estimates the metabolic health degree by weighting, with the second parameter, the water balance and a factor that is not used for estimating the water balance among the factor that determines the total energy expenditure. For example, the water balance is determined by weighting the physical activity level, the athlete level, and the age among the factor that determines the total energy expenditure described above. At this time, for example, as shown in the following formula (5), the health degree estimation unit 21 determines the metabolic health degree by weighting, with the second parameter, the fat free mass and the body fat percentage, and the water balance among the factor that determines the total energy expenditure. Weighting coefficients c1, c2, and c3 in the following formula (5) are the second parameter. For example, the weighting coefficients c1, c2, and c3 are positive numbers. It is assumed that the health degree estimation unit 21 holds information on the second parameter in advance.

$$\text{Metabolic health degree} = c1*WT + c2*FFM - c3*BFP \qquad (5)$$

For example, the health degree estimation unit 21 may hold one or the plurality of pieces of first physical information and the first water metabolism index of the subject to be stored that are stored in the storage unit 11 and information on the second parameter determined based on the real metabolic health degree set according to the first water metabolism index. The real metabolic health degree can be determined according to the ratio of the first water metabolism index to a target water metabolism index determined in advance according to the gender and age. For example, when the first water metabolism index exceeds the target water metabolism index, the real metabolic health degree increases, and when the first water metabolism index falls below the target water metabolism index, the real metabolic health degree decreases. At this time, the second parameter for obtaining the metabolic health degree by weighting each of the physical information and the water metabolism index is determined by regression analysis using the first physical information and the first water metabolism index as explanatory variables and the actual metabolic health degree as a target variable. The health degree estimation unit 21 may hold the second parameter determined as described above in advance.

It can be considered that the higher the metabolic health degree, the higher the health degree. For example, in the case where the target metabolic health degree is determined in advance according to the gender and the age, when the metabolic health degree calculated by the above formula (5) is equal to or higher than the target metabolic health degree, it can be regarded as being healthy.

FIG. 6 illustrates hardware configurations of the model determination device 10, the water metabolism index estimation device 1, and the health degree estimation device 2 having the above-described configurations. The model determination device 10, the water metabolism index estimation device 1, and the health degree estimation device 2 each include a processor 81, a memory 82, and an interface 83. The processor 81, the memory 82, and the interface 83 are connected to each other by a bus 80. Functions of each unit of the model determination device 10, the water metabolism index estimation device 1, and the health degree estimation device 2 are implemented by software, firmware, or a combination of software and firmware. The software and the firmware are described as programs and stored in the memory 82. When the processor 81 reads and executes the program stored in the memory 82, the functions of the above-described units are implemented. That is, the memory 82 stores a program for executing processing of each unit of the model determination device 10, the water metabolism index estimation device 1, and the health degree estimation device 2.

The memory 82 includes, for example, a nonvolatile or volatile semiconductor memory such as a random access memory (RAM), a read-only memory (ROM), a flash memory, an erasable programmable read only memory (EPROM), or an electrically erasable and programmable read-only memory (EEPROM), a magnetic disk, a flexible disk, an optical disk, a compact disc (CD), a mini disk, or a digital versatile disc (DVD).

The model determination device 10 is connected to the water metabolism index estimation device 1 via the interface 83. The water metabolism index estimation device 1 is connected to an input and output device, specifically, the body composition meter, the heart rate meter, or the like, via the interface 83. The health degree estimation device 2 is connected to, via the interface 83, a display device, or the like, that displays the metabolic health degree. The interface 83 includes an interface module conforming to one or a plurality of standards depending on a connection destination.

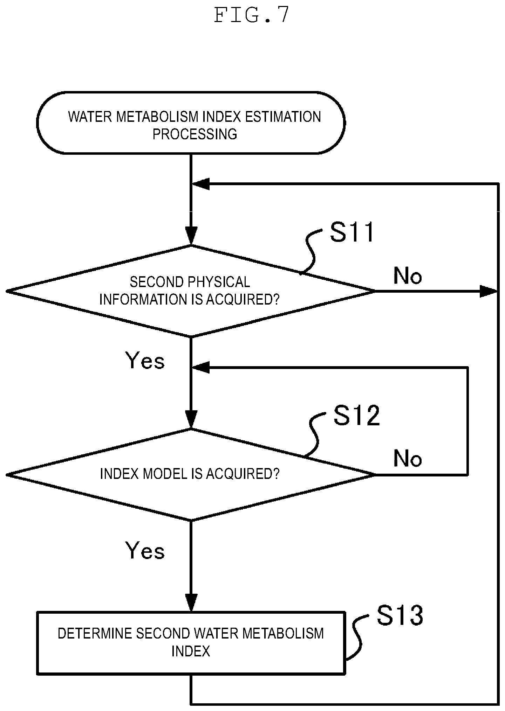

Water metabolism index estimation processing performed by the water metabolism index estimation unit 14 provided in the water metabolism index estimation device 1 having the above configuration will be described below with reference to FIG. 7. The water metabolism index estimation device 1 performs the water metabolism index estimation processing on the subject to be estimated at a timing independent of the processing in which the model determination device 10 determines the index model. The water metabolism index estimation device 1 starts the processing illustrated in FIG. 7 when operating. While the second physical information of the subject to be estimated is not acquired (step S11; No), the water metabolism index estimation unit 14 repeats the processing of step S11.

When the second physical information of the subject to be estimated is acquired (step S11; Yes), the water metabolism index estimation unit 14 acquires the index model, specifically, the first parameter from the determination unit 12 (step S12). While the first parameter which is the index model is not acquired (step S12; No), the water metabolism index estimation unit 14 repeats the processing of step S11.

When the first parameter which is the index model is acquired (step S12; Yes), the water metabolism index estimation unit 14 applies the second physical information to the index model, specifically, weights each piece of the second physical information with the first parameter to determine the second water metabolism index (step S13). When the processing of step S13 ends, the above-described processing is repeated from step S11.

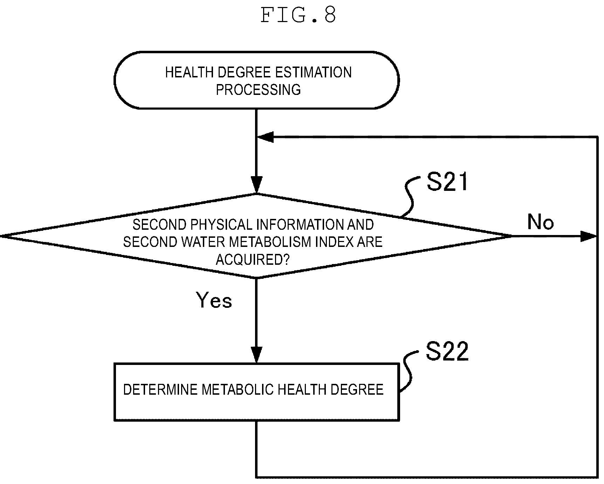

Health degree estimation processing performed by the health degree estimation unit 21 provided in the health degree estimation device 2 having the above configuration will be described below with reference to FIG. 8. The health degree estimation device 2 starts the processing in FIG. 8 when operating. The health degree estimation unit 21 acquires the second physical information from the physical information acquisition unit 13 and acquires the second water metabolism index from the water metabolism index estimation unit 14 (step S21). While at least one of the second physical information and the second water metabolism index is not acquired (step S21; No), the health degree estimation unit 21 repeats the processing of step S21.

When the second physical information and the second water metabolism index are acquired (step S21; Yes), the health degree estimation unit 21 weights each of the water balance and the second physical information with the second parameter to determine the metabolic health degree (step S22). When the processing of step S22 ends, the above-described processing is repeated from step S21.

As described above, the water metabolism index estimation device 1 according to Embodiment 1 determines, based on the first water metabolism index and the first physical information of the subject to be stored, the first parameter which is a weighting coefficient for determining the water metabolism index by weighting the physical information, and weights each piece of the second physical information of the subject to be estimated with the first parameter to estimate the second water metabolism index of the subject to be estimated. Accordingly, the second water metabolism index indicating the degree of water metabolism of the subject to be estimated is obtained only by inputting the physical feature of the subject to be estimated to the water metabolism index estimation device 1.

The water metabolism index estimation device 1 determines the first parameter based on the first water metabolism index and the first physical information of the subject to be stored that are composed of the data obtained from the DLW database, and determines the second water metabolism index by weighting, with the first parameter, the second physical information indicating the physical feature of the subject to be estimated. Therefore, it is not necessary to measure the water balance WT based on the doubly-labelled water method every time the second water metabolism index is determined for the subject to be estimated, and the second water metabolism index can be simply determined.

(Embodiment 2)

The configuration of the water metabolism index estimation device 1 is not limited to the above-described example. A water metabolism index estimation device 3 that determines a second water metabolism index based on the environmental state around a subject to be estimated in addition to a physical feature of the subject to be estimated will be described in Embodiment 2.

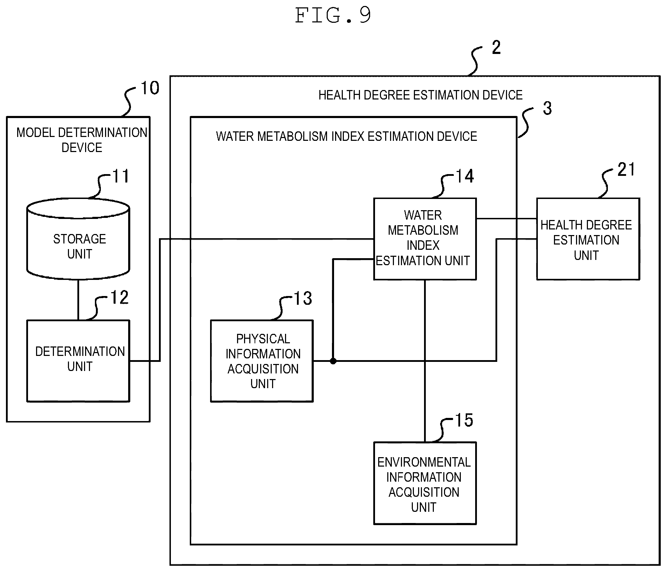

The water metabolism index estimation device 3 illustrated in FIG. 9 further includes an environmental information acquisition unit 15 that acquires second environmental information indicating the environmental state around the subject to be estimated in addition to the configuration of the water metabolism index estimation device 1 according to Embodiment 1.

A DLW database also stores information indicating the environmental state around a subject to be measured. The storage unit 11 stores, based on data obtained from the DLW database, a first physical information of each subject to be stored, a first water metabolism index of each subject to be stored, and one or a plurality of pieces of first environmental information numerically indicating the environmental state around each subject to be stored, in association with each other. The environmental state includes at least one of the temperature, the humidity, the latitude, the altitude, and the human development index (HDI). The temperature and the humidity are, for example, an average value of the temperature and an average value of the humidity over a measurement period during which analysis based on a doubly-labelled water method is performed. The latitude and the altitude indicate the latitude and the altitude, respectively, of the location where analysis based on the doubly-labelled water method is performed. The human development index is based on an indicator calculated for each country by the United Nations Development Programme according to life expectancy, education, literacy and income indices, and is determined for each country for which analysis based on the doubly-labelled water method is performed. For example, the human development index used in Embodiment 2 is one of the following values: 1 indicating that the index is high, 2 indicating that the index is medium, and 3 indicating that the index is low.

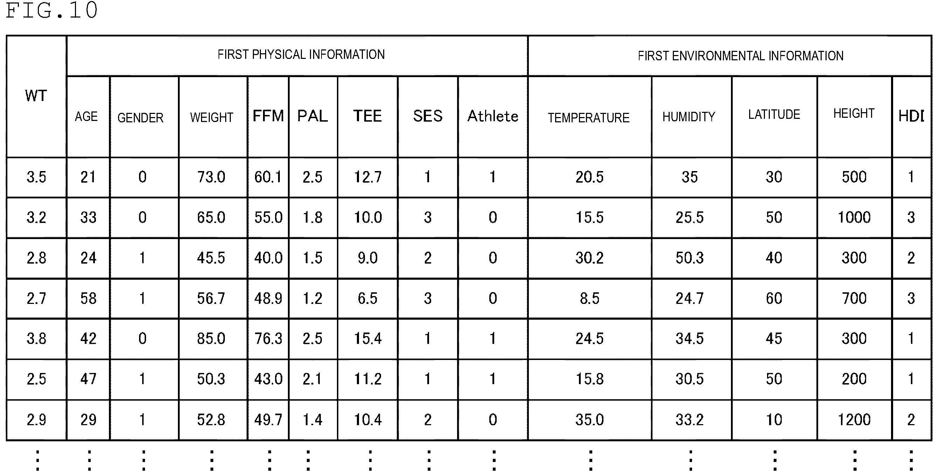

As illustrated in FIG. 10, the storage unit 11 stores the first physical information, the first water metabolism index, and the first environmental information of each subject to be stored, in association with each other. In the example in FIG. 10, the first environmental information includes the temperature, the humidity, the latitude, the altitude, and the human development index. In FIG. 10, the human development index is illustrated as HDI. Each record in the table illustrated in FIG. 10 is data corresponding to each subject to be stored.

The determination unit 12 acquires the water balance WT, the first physical information, and the first environmental information of each subject to be stored that are stored in the storage unit 11. The determination unit 12 determines a plurality of first parameters for weighting each of the physical information and the environmental information to obtain the water metabolism index, and sends the first parameters to the water metabolism index estimation device 3. Specifically, the determination unit 12 determines the first parameters by multiple regression analysis using each of the first physical information and the first environmental information as an explanatory variable and the first water metabolism index as an objective variable. The determination unit 12 sends the first parameters to the water metabolism index estimation unit 14 of the water metabolism index estimation device 3.

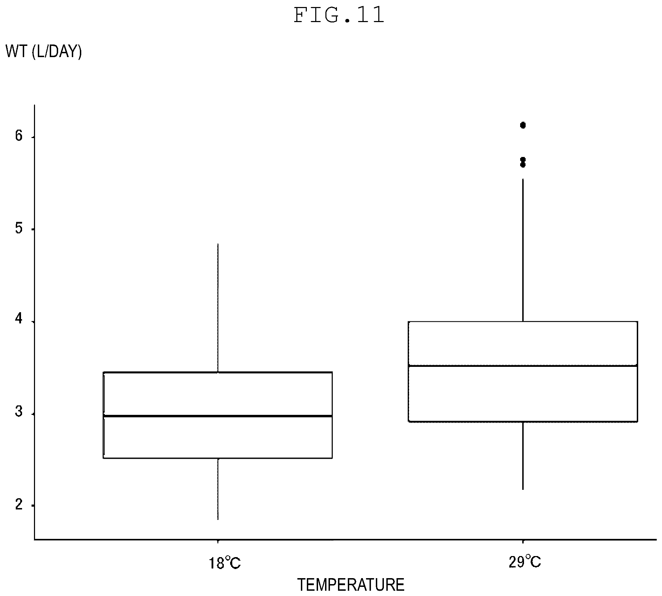

It is preferable that the determination unit 12 determines the first parameter by performing multiple regression analysis using, as explanatory variables, the first environmental information and the first physical information having a high correlation with the first water metabolism index among the first physical information. FIG. 11 illustrates an example of the relationship between the temperature (unit: °C), which is an example of the first environmental information, and the water balance WT (unit: L/day). The vertical axis represents the water balance WT. FIG. 11 is a box plot illustrating the variation in the water balance WT for each temperature. The left side of the drawing is a graph illustrating the water balance WT of each subject to be stored when the temperature is 18°C. The right side of the drawing is a graph illustrating the water balance WT of each subject to be stored when the temperature is 29°C. As illustrated in FIG. 11, the box corresponding to the water balance WT when the temperature is 29°C is located higher than the box corresponding to the water balance WT when the temperature is 18°C. That is, the temperature has a positive correlation with the water balance WT.

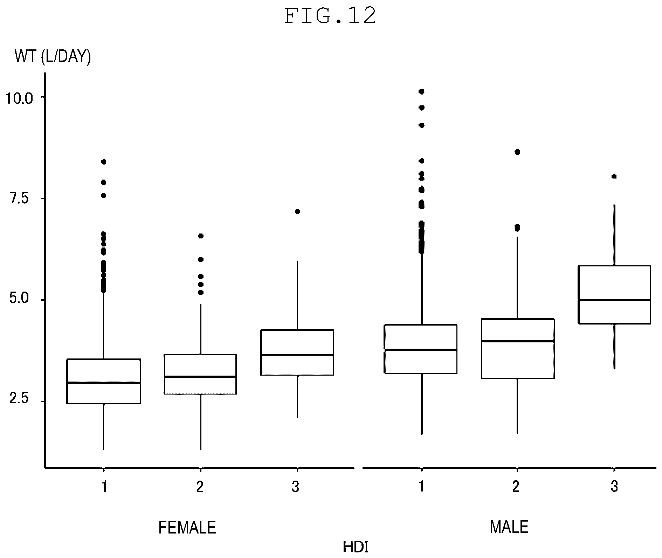

FIG. 12 illustrates an example of the relationship between the human development index, which is an example of the first environmental information, and the water balance WT (unit: L/day). The vertical axis represents the water balance WT. FIG. 12 is a box plot illustrating the variation in the water balance WT for each human development index. Graphs illustrating the water balance WT of each of the subjects to be stored that correspond to a woman with HDI = 1, a woman with HDI = 2, a woman with HDI = 3, a man with HDI = 1, a man with HDI = 2, and a man with HDI = 3 are displayed in this order from the left of the drawing. As illustrated in FIG. 12, the higher the value of HDI, the higher the position of the box corresponding to the water balance WT. That is, the HDI has a positive correlation with the water balance WT.

In order to accurately determine the water balance WT, it is preferable to perform the multiple regression analysis using the first physical information and the first environmental information having a high correlation with the water balance WT. As an example, the determination unit 12 determines the first parameter by multiple regression analysis using, as explanatory variables, the first physical information including the gender, the age, the weight, the physical activity level, and the athlete level, and the first environmental information including the temperature, the humidity, the human development index, and the altitude, and using the first water metabolism index as an objective variable.

The model determination device 10 illustrated in FIG. 9 is implemented by the data acquired from the DLW database as described above, determines, based on the water balance WT, the first physical information, and the first environmental information that are stored in the storage unit 11, the first parameter for weighting the physical information and the environmental information to determine the water metabolism index, and sends the first parameter as the index model to the water metabolism index estimation device 3.

Details of the water metabolism index estimation device 3 that acquires the first parameter as the index model from the model determination device 10 described above and determines the water metabolism index of the subject to be estimated will be described below. As in Embodiment 1, the subject to be estimated is an individual different from the subject to be stored that is associated with the data stored in the storage unit 11 of the model determination device 10, and is an individual who is a subject of water metabolism index estimation processing.

The environmental information acquisition unit 15 acquires the environmental state around the subject to be estimated. Specifically, the environmental information acquisition unit 15 acquires one or a plurality of pieces of second environmental information numerically indicating the environmental state around the subject to be estimated, and sends the acquired second environmental information to the water metabolism index estimation unit 14. The environmental state acquired by the environmental information acquisition unit 15 includes the environmental state indicated by the first environmental information stored in the storage unit 11.

For example, the environmental information acquisition unit 15 acquires position information of the subject to be estimated from a global positioning system (GPS) receiver worn by the subject to be estimated, and determines the human development index based on the position information. It is assumed that the environmental information acquisition unit 15 holds in advance information on the association between the position information and the human development index determined for each country. The environmental information acquisition unit 15 uses the GPS altitude included in the position information of the subject to be estimated as the altitude constituting the second environmental information.

The environmental information acquisition unit 15 acquires, based on the position information of the subject to be estimated, weather data at the point where the subject to be estimated is located from an external device, and uses the temperature and humidity at the point obtained from the weather data as the temperature and humidity constituting the second environmental information.

The water metabolism index estimation unit 14 weights, with the first parameter acquired from the determination unit 12, each of the second physical information acquired from the physical information acquisition unit 13 and the second environmental information acquired from the environmental information acquisition unit 15, and determines the water balance as the second water metabolism index indicating the degree of water metabolism of the subject to be estimated. The water metabolism index estimation unit 14 sends the obtained second water metabolism index to the health degree estimation unit 21.

The water metabolism index estimation unit 14 determines the water balance WT (unit: mL/day) by weighting, with the first parameter, the second physical information including the gender, the age, the weight, the physical activity level, and the athlete level, and the second environmental information including the temperature, the humidity, the human development index, and the altitude, based on the following formula (6). In the following formula (6), the weight is represented by kg, the temperature is represented by degrees Celsius, the humidity is represented by percentage, and the altitude is represented by meter. Weighting coefficients $a_1$, $a_2$, $a_3$, $a_4$, $a_5$, $a_6$, $a_7$, $a_8$, and $a_9$ in the following formula (6) are the first parameter. For example, the weighting coefficients $a_1$, $a_2$, $a_3$, $a_4$, $a_5$, $a_6$, $a_7$, $a_8$, and $a_9$ are positive numbers.

$$WT = a_1 * PAL + a_2 * weight + a_3 * gender + a_4 * Athlete - a_5 * age + a_6 * temperature + a_7 * humidity + a_8 * HDI + a_9 * altitude \tag{6}$$

As an example, the water balance WT is determined by weighting the second physical information and the second environmental information with the first parameter, as shown in the following formula (7).

$$WT = 1020 * PAL + 14.26 * weight + 395.5 * gender + 30.39 * temperature + 6.129 * humidity + 896.0 * Athlete + 229.2 * HDI + 0.2654 * altitude - 13.05 * age \tag{7}$$

The second physical information to be weighted is assumed to include a numerical value obtained by performing a calculation on the second physical information, for example, a power of the second physical information. Similarly, the second environmental information to be weighted is assumed to include a numerical value obtained by performing a calculation on the second environmental information, for example, a power of the second environmental information. The water balance WT is not limited to the above formula (7), and may be determined by weighting the second physical information and the second environmental information with the first parameter, as shown in the following formula (8). In the following formula (8), $(age)^2$ represents the square of age, and $(temperature)^2$ represents the square of temperature. In the following formula (8), -713.1 is a constant.

$$WT = 1076 * PAL + 14.34 * weight + 374.9 * gender + 5.823 * humidity + 1070 * Athlete + 104.6 * HDI + 0.4726 * altitude - 0.3529 * (age)^2 + 24.78 * age + 1.865 * (temperature)^2 - 19.66 * temperature - 713.1 \tag{8}$$

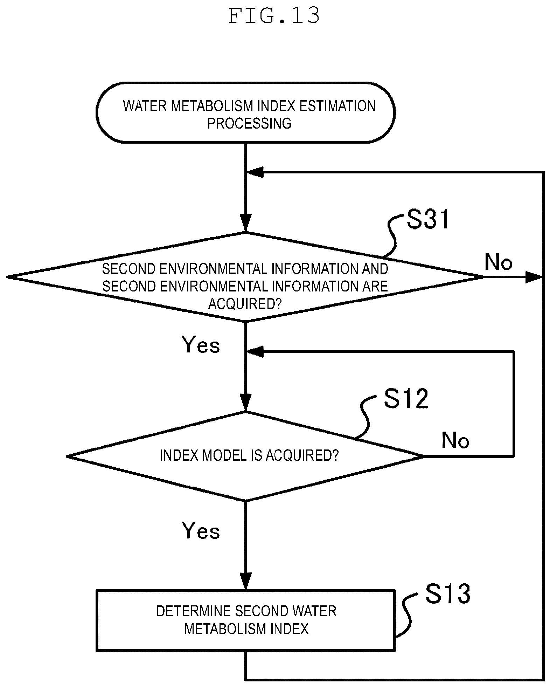

The hardware configuration of the water metabolism index estimation device 3 having the above configuration is the same as that of the water metabolism index estimation device 1 according to Embodiment 1. Water metabolism index estimation processing performed by the water metabolism index estimation unit 14 provided in the water metabolism index estimation device 3 having the above configuration will be described below with reference to FIG. 13. The water metabolism index estimation device 3 performs water metabolism index estimation processing on the subject to be estimated at a timing independent of the processing in which the model determination device 10 determines the index model. The water metabolism index estimation device 3 starts the processing illustrated in FIG. 13 when operating. While at least one of the second physical information and the second environmental information of the subject to be estimated is not acquired (step S31; No), the water metabolism index estimation unit 14 repeats the processing of step S31.

When the second physical information and the second environmental information of the subject to be estimated are acquired (step S31; Yes), the water metabolism index estimation unit 14 acquires the index model, specifically, the first parameter from the determination unit 12 (step S12). Processing after step S12 is the same as the processing performed by the water metabolism index estimation device 1 according to Embodiment 1 illustrated in FIG. 7.

As described above, the water metabolism index estimation device 3 according to Embodiment 2 determines, based on the first water metabolism index, the first physical information, and the first environmental information of the subject to be stored, the first parameter which is a weighting coefficient for determining the water metabolism index by weighting the physical information and the environmental information, and estimates the second water metabolism index of the subject to be estimated by weighting, with the first parameter, each of the second physical information and the second environmental information of the subject to be estimated. Accordingly, the second water metabolism index indicating the degree of water metabolism of the subject to be estimated is obtained only by inputting the physical feature of the subject to be estimated and the environmental state around the subject to be estimated to the water metabolism index estimation device 3.

As in Embodiment 1, it is not necessary to measure the water balance WT based on the doubly-labelled water method every time the second water metabolism index is determined for the subject to be estimated, and the second water metabolism index can be simply determined. By determining the second water metabolism index based on the environmental state around the subject to be estimated in addition to the physical feature of the subject to be estimated, the second water metabolism index can be obtained more accurately.

The present disclosure is not limited to the above example. The first physical information and the first environmental information that are stored in the storage unit 11 are not limited to the examples described above, and may be any data as long as they are data obtained from the DLW database or data derived from the DLW database.

The method for determining the first parameter in the determination unit 12 is not limited to the above example. As an example, the determination unit 12 may determine the first parameter by single regression analysis using one type of first physical information as an explanatory variable and the water balance WT as an objective function.

As another example, the determination unit 12 learns the first physical information and the water balance WT that are stored in the storage unit 11, and determines an index model, which is a neural network model that receives the physical information as an input, and outputs the water metabolism index. Specifically, the determination unit 12 learns learning data including the first physical information and the water balance WT, uses the physical information as an input value, uses the water balance as an output value, and generates a neural network model having an input layer, an intermediate layer, and an output layer. The determination unit 12 adjusts the weight between the input layer and the intermediate layer, the weight between the intermediate layers, and the weight between the intermediate layer and the output layer based on the learning data including the first physical information and the water balance WT.

As another example, the determination unit 12 learns the first physical information, the water balance WT, and the first environmental information that are stored in the storage unit 11, and determines an index model, which is a neural network model that receives the physical information and the environmental information as inputs, and outputs the water metabolism index. Specifically, the determination unit 12 learns learning data including the first physical information, the water balance WT, and the first environmental information, uses the physical information and the environmental information as input values, uses the water balance as an output value, and generates a neural network model having an input layer, an intermediate layer, and an output layer. The determination unit 12 adjusts the weight between the input layer and the intermediate layer, the weight between the intermediate layers, and the weight between the intermediate layer and the output layer based on the learning data including the first physical information, the water balance WT, and the first environmental information.

The index indicating the degree of water metabolism is not limited to the water balance. As an example, the water metabolism ratio obtained by dividing the water balance by total body water may be used as the water metabolism index.

The physical information acquisition unit 13 may acquire the second physical information which is the measurement value of the physical feature of the subject to be estimated and other pieces of the second physical information which is the estimated value estimated from the measurement value. As an example, the physical information acquisition unit 13 may estimate the body fat percentage based on the height and the weight acquired from the body composition meter.

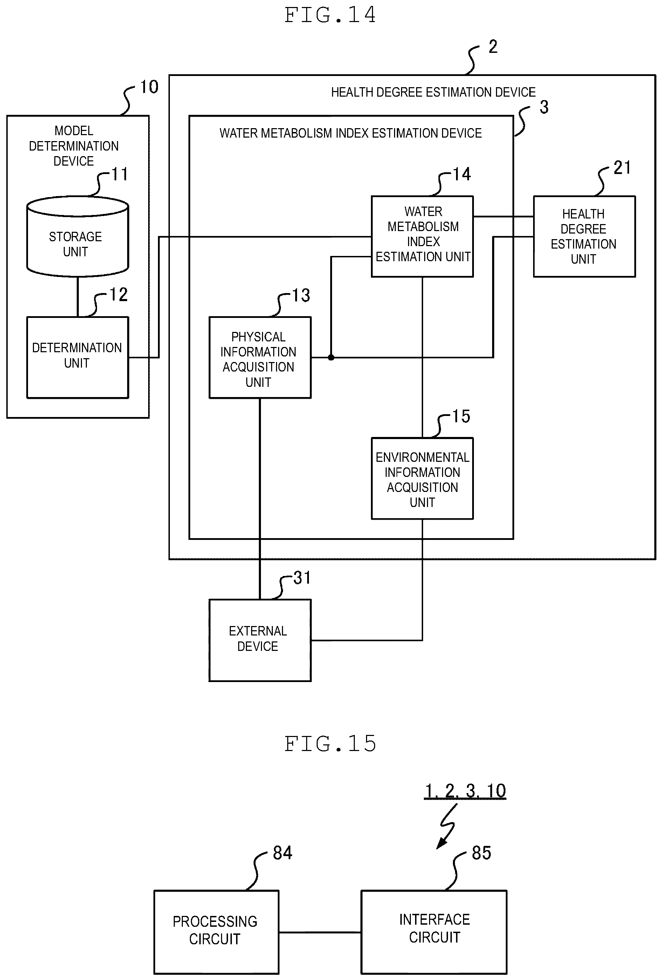

As illustrated in FIG. 14, the physical information acquisition unit 13 and the environmental information acquisition unit 15 may acquire the heart rate of the subject to be estimated, position information of the subject to be estimated, and the like, from an external device 31, for example, a smart watch, capable of measuring biological information of the subject to be estimated and the environmental state around the subject to be estimated.

The method for estimating the metabolic health degree is not limited to the above example. The metabolic health degree estimated based on the first physical information and the water balance WT of each subject to be stored may be stored in the storage unit 11 based on any one of the above formulae (4) and (5). In this case, the health degree estimation unit 21 may determine, based on the association of the first physical information, the water balance WT, and the metabolic health degree that are stored in the storage unit 11, a health degree model for deriving the metabolic health degree from the physical information and the water metabolism index. The health degree estimation unit 21 may determine the metabolic health degree by applying the second physical information to the health degree model.

The hardware configuration and the flowchart described above are examples, and any change and modification can be made. The core part that performs control processing and including the processor 81, the memory 82, and the interface 83 can be implemented by using a normal computer system, without using a dedicated system. For example, a computer program for executing the above-described operations may be stored and distributed in a computer-readable recording medium (such as a flexible disk, a compact disc-read only memory (CD-ROM), and a digital versatile disc-read only memory (DVD-ROM)), and the model determination device 10, the water metabolism index estimation devices 1 and 3, and the health degree estimation device 2 that execute the above-described processing may be implemented by installing the computer program on a computer. The model determination device 10, the water metabolism index estimation device 1 and 3, and the health degree estimation device 2 may be implemented by storing the above-described computer program in a storage device provided in a server device on a communication network and downloading the program to a normal computer system.

When the functions of the model determination device 10, the water metabolism index estimation devices 1 and 3, and the health degree estimation device 2 are implemented by sharing between an operating system (OS) and an application program or cooperation between the OS and the application program, only the application program portion may be stored on a recording medium or a storage device.

A computer program may be superimposed on a carrier wave and distributed via a communication network. For example, the computer program may be posted on a bulletin board system (BBS) on a communication network, and the computer program may be distributed via the communication network. Then, the computer program may be activated and executed under control of the OS in the same manner as other application programs, thereby enabling the above-described processing to be performed.

The hardware configurations of the model determination device 10, the water metabolism index estimation device 1, 3, and the health degree estimation device 2 are not limited to the above example. The model determination device 10, the water metabolism index estimation device 1, 3, and the health degree estimation device 2 may be implemented by a processing circuit 84 as illustrated in FIG. 15. The processing circuit 84 is connected to an input and output device, or the like, via an interface circuit 85.

When the processing circuit 84 is dedicated hardware, the processing circuit 84 includes, for example, a single circuit, a composite circuit, a processor, an application specific integrated circuit (ASIC), a field programmable gate array (FPGA), or a combination thereof. Each unit of the model determination device 10, the water metabolism index estimation device 1, 3, and the health degree estimation device 2 may be implemented by the individual processing circuit 84 or may be implemented by the common processing circuit 84.

A part of the functions of the model determination device 10, the water metabolism index estimation devices 1 and 3, and the health degree estimation device 2 may be implemented by dedicated hardware, and the other parts may be implemented by software or firmware. For example, in the water metabolism index estimation device 1 according to Embodiment 1, the physical information acquisition unit 13 may be implemented by the processing circuit 84 illustrated in FIG. 15, and the water metabolism index estimation unit 14 may be implemented by the processor 81 illustrated in FIG. 6 reading and executing a program stored in the memory 82.

The embodiments described above are for describing the present disclosure and do not limit the scope of the present disclosure. That is, the scope of the present disclosure is defined by the claims, not by the embodiments. Various modifications made within the scope of the claims and the meaning of the invention equivalent thereto are considered to be within the scope of the present disclosure.

The present application is based on Japanese Patent Application No. 2022-132055 filed on August 22, 2022. The description, claims, and drawings of Japanese Patent Application No. 2022-132055 are hereby incorporated by reference herein in its entirety.

Reference Signs List

1, 3    water metabolism index estimation device

2     health degree estimation device
10    model determination device
11    storage unit
12    determination unit
13    physical information acquisition unit

14    water metabolism index estimation unit
15    environmental information acquisition unit
21    health degree estimation unit
31    external device
80    bus
81    processor
82    memory
83    interface
84    processing circuit
85    interface circuit

**Claims**

1.    A model determination device comprising:

a storage unit configured to store, for a plurality of subjects to be stored, one or a plurality of pieces of first physical information numerically indicating a physical feature of the subject to be stored and a first water metabolism index indicating the degree of water metabolism of the subject to be stored in a predetermined period in association with each other; and
a determination unit configured to determine, based on the association of the first physical information and the first water metabolism index of each of the subjects to be stored, an index model for deriving, from physical information indicating a physical feature, the water metabolism index indicating the degree of water metabolism.

2.    The model determination device according to claim 1, wherein
the determination unit determines, by regression analysis using the first physical information as an explanatory variable and the first water metabolism index as an objective function, one or a plurality of first parameters for weighting each piece of the physical information to obtain the water metabolism index.

3.    The model determination device according to claim 1, wherein
the determination unit learns the first physical information and the first water metabolism index, and determines the index model, which is a neural network model that receives the physical information as an input, and outputs the water metabolism index.

4.    The model determination device according to claim 1, wherein
the storage unit stores, for the plurality of subjects to be stored, the first physical information, the first water metabolism index, and one or a plurality of pieces of first environmental information numerically indicating the environmental state around the subject to be stored, in association with each other.

5.    The model determination device according to claim 4, wherein
the determination unit determines, based on the association of the first physical information, the first water metabolism index, and the first environmental information of each of the subjects to be stored, the index model for deriving the water metabolism index from the physical information and environmental information numerically indicating an environmental state.

6.    The model determination device according to claim 5, wherein
the determination unit determines, by regression analysis using the first physical information and the first environmental information as explanatory variables and using the first water metabolism index as an objective function, a plurality of first parameters for weighting each of the physical information and the environmental information to obtain the water metabolism index.

7.    The model determination device according to claim 6, wherein
the determination unit determines the plurality of first parameters by multiple regression analysis using, as explanatory variables, the first physical information including the physical activity level, the weight, the gender, the athlete level, and the age, and the first environmental information including the temperature, the humidity, and the human development index, and using the first water metabolism index as an objective function.

8.    The model determination device according to claim 5, wherein
the determination unit learns the first physical information, the first water metabolism index, and the first environmental information, and determines the index model, which is a neural network model that receives the physical information and the environmental information as inputs, and outputs the water metabolism index.

9. The model determination device according to any one of claims 1 to 8, wherein
   the storage unit stores the first water metabolism index determined based on a doubly-labelled water method.

10. A water metabolism index estimation device comprising:

   a physical information acquisition unit configured to acquire one or a plurality of pieces of second physical information numerically indicating a physical feature of a subject to be estimated; and
   a water metabolism index estimation unit configured to acquire an index model for deriving, from physical information indicating a physical feature, the water metabolism index indicating the degree of water metabolism, the index model being determined by associating, for a plurality of subjects to be stored, one or a plurality of pieces of first physical information numerically indicating a physical feature of the subject to be stored with a first water metabolism index indicating the degree of water metabolism of the subject to be stored in a predetermined period, and determine, by applying the second physical information to the index model, a second water metabolism index indicating the degree of water metabolism of the subject to be estimated.

11. The water metabolism index estimation device according to claim 10, wherein
   the physical information acquisition unit acquires one or a plurality of pieces of the second physical information which are measurement values of the physical feature of the subject to be estimated, and one or a plurality of other pieces of the second physical information estimated from the second physical information.

12. The water metabolism index estimation device according to claim 10, wherein
   the physical information acquisition unit acquires one or a plurality of pieces of the second physical information which are measurement values of the physical feature of the subject to be estimated, and one or a plurality of other pieces of the second physical information whose values are determined in advance.

13. The water metabolism index estimation device according to any one of claims 10 to 12, wherein
   the physical feature includes at least one of the gender, the age, the weight, the fat free mass, the physical activity level, the total energy expenditure, the socioeconomic status, and the athlete level indicating the daily exercise intensity.

14. The water metabolism index estimation device according to any one of claims 10 to 13, further comprising:

   an environmental information acquisition unit configured to acquire one or a plurality of pieces of second environmental information numerically indicating the environmental state around the subject to be estimated, wherein
   the water metabolism index estimation unit acquires the index model for deriving the water metabolism index from the physical information and environmental information indicating the environmental state, the index model being determined by associating, for the plurality of subjects to be stored, the first physical information, the first water metabolism index, and one or a plurality of pieces of first environmental information numerically indicating the environmental state around the subject to be stored, and determines the second water metabolism index by applying the second physical information and the second environmental information to the index model.

15. The water metabolism index estimation device according to claim 14, wherein
   the environmental information acquisition unit acquires one or a plurality of pieces of the second environmental information which are measurement values of the environmental state around the subject to be estimated, and one or a plurality of other pieces of the second environmental information estimated from the second environmental information.

16. The water metabolism index estimation device according to claim 14, wherein
   the environmental information acquisition unit acquires one or a plurality of pieces of the second environmental information which are measurement values of the environmental state around the subject to be estimated, and one or a plurality of other pieces of the second environmental information whose values are determined in advance.

17. The water metabolism index estimation device according to any one of claims 14 to 16, wherein
   the environmental state includes at least any one of the temperature, the humidity, the latitude, the altitude, and the human development index.

18. A health degree estimation device comprising:

the water metabolism index estimation device according to any one of claims 10 to 17; and
a health degree estimation unit configured to determine the metabolic health degree by weighting each of the second water metabolism index and the second physical information with a predetermined second parameter.

19. A model determination method comprising:

determining an index model for deriving, from physical information indicating a physical feature, the water metabolism index indicating the degree of water metabolism by associating, for a plurality of subjects to be stored, one or a plurality of pieces of first physical information numerically indicating a physical feature of the subject to be stored with a first water metabolism index indicating the degree of water metabolism of the subject to be stored in a predetermined period.

20. A water metabolism index estimation method comprising:

acquiring one or a plurality of pieces of second physical information numerically indicating a physical feature of a subject to be estimated; and
acquiring an index model for deriving, from physical information indicating a physical feature, the water metabolism index indicating the degree of water metabolism, the index model being determined by associating, for a plurality of subjects to be stored, one or a plurality of pieces of first physical information numerically indicating a physical feature of the subject to be stored with a first water metabolism index indicating the degree of water metabolism of the subject to be stored in a predetermined period, and determining, by applying the second physical information to the index model, a second water metabolism index indicating the degree of water metabolism of the subject to be estimated.

21. A health degree estimation method comprising:

acquiring one or a plurality of pieces of second physical information numerically indicating a physical feature of a subject to be estimated;
acquiring an index model for deriving, from physical information indicating a physical feature, the water metabolism index indicating the degree of water metabolism, the index model being determined by associating, for a plurality of subjects to be stored, one or a plurality of pieces of first physical information numerically indicating a physical feature of the subject to be stored with a first water metabolism index indicating the degree of water metabolism of the subject to be stored in a predetermined period, and determining, by applying the second physical information to the index model, a second water metabolism index indicating the degree of water metabolism of the subject to be estimated; and
determining the metabolic health degree by weighting each of the second water metabolism index and the second physical information with a predetermined second parameter.

22. A program for causing a computer to function as:

a storage unit configured to store, for a plurality of subjects to be stored, one or a plurality of pieces of first physical information numerically indicating a physical feature of the subject to be stored and a first water metabolism index indicating the degree of water metabolism of the subject to be stored in a predetermined period in association with each other; and
a determination unit configured to determine, based on the association of the first physical information and the first water metabolism index of each of the subjects to be stored, an index model for deriving, from physical information indicating a physical feature, the water metabolism index indicating the degree of water metabolism.

23. A program for causing a computer to function as:

a physical information acquisition unit configured to acquire one or a plurality of pieces of second physical information numerically indicating a physical feature of a subject to be estimated; and
a water metabolism index estimation unit configured to acquire an index model for deriving, from physical information indicating a physical feature, the water metabolism index indicating the degree of water metabolism, the index model being determined by associating, for a plurality of subjects to be stored, one or a plurality of pieces of first physical information numerically indicating a physical feature of the subject to be stored with a first water metabolism index indicating the degree of water metabolism of the subject to be stored in a predetermined period, and determine, by applying the second physical information to the index model, a second water metabolism index indicating the degree of water metabolism of the subject to be estimated.

24. A program for causing a computer to function as:

a physical information acquisition unit configured to acquire one or a plurality of pieces of second physical information numerically indicating a physical feature of a subject to be estimated;

a water metabolism index estimation unit configured to acquire an index model for deriving, from physical information indicating a physical feature, the water metabolism index indicating the degree of water metabolism, the index model being determined by associating, for a plurality of subjects to be stored, one or a plurality of pieces of first physical information numerically indicating a physical feature of the subject to be stored with a first water metabolism index indicating the degree of water metabolism of the subject to be stored in a predetermined period, and determine, by applying the second physical information to the index model, a second water metabolism index indicating the degree of water metabolism of the subject to be estimated; and

a health degree estimation unit configured to determine the metabolic health degree by weighting each of the second water metabolism index and the second physical information with a predetermined second parameter.

# FIG.1

# FIG.2

| WT | FIRST PHYSICAL INFORMATION | | | | | | | |
|---|---|---|---|---|---|---|---|---|
| | AGE | GENDER | WEIGHT | FFM | PAL | TEE | SES | Athlete |
| 3.5 | 21 | 0 | 73.0 | 60.1 | 2.5 | 12.7 | 1 | 1 |
| 3.2 | 33 | 0 | 65.0 | 55.0 | 1.8 | 10.0 | 3 | 0 |
| 2.8 | 24 | 1 | 45.5 | 40.0 | 1.5 | 9.0 | 2 | 0 |
| 2.7 | 58 | 1 | 56.7 | 48.9 | 1.2 | 6.5 | 3 | 0 |
| 3.8 | 42 | 0 | 85.0 | 76.3 | 2.5 | 15.4 | 1 | 1 |
| 2.5 | 47 | 1 | 50.3 | 43.0 | 2.1 | 11.2 | 1 | 1 |
| 2.9 | 29 | 1 | 52.8 | 49.7 | 1.4 | 10.4 | 2 | 0 |
| ⋮ | ⋮ | ⋮ | ⋮ | ⋮ | ⋮ | ⋮ | ⋮ | ⋮ |

FIG.3

FIG.4

## FIG.5

WT (L/DAY)

|   |   |   |   |
|---|---|---|---|
| 1 | 0 | 1 | 0 |
| (FEMALE ATHLETE) | (FEMALE NON-ATHLETE) | (MALE ATHLETE) | (MALE NON-ATHLETE) |

Athlete

## FIG.6

1, 2, 10

81 PROCESSOR

80

82 MEMORY

83 INTERFACE

FIG.7

```
   ╭─────────────────────────────────────╮
   │  WATER METABOLISM INDEX ESTIMATION  │
   │             PROCESSING              │
   ╰─────────────────────────────────────╯
                     │
                     ▼
                              ⟋S11
              ◇─────────────────◇           No
            ◇   SECOND PHYSICAL   ◇ ─────────────▶
              ◇ INFORMATION IS ACQUIRED? ◇
              ◇─────────────────◇
                     │ Yes
                     ▼
                              ⟋S12
              ◇─────────────────◇           No
            ◇  INDEX MODEL IS ACQUIRED?  ◇ ───────
              ◇─────────────────◇
                     │ Yes
                     ▼
   ┌─────────────────────────────────────┐  ⟋S13
   │  DETERMINE SECOND WATER METABOLISM   │
   │               INDEX                  │
   └─────────────────────────────────────┘
```

FIG.8

```
        ┌──────────────────────────────┐
        │  HEALTH DEGREE ESTIMATION    │
        │         PROCESSING           │
        └──────────────────────────────┘
                      │
                      ▼
            ╱────────────────────╲                    S21
          ╱  SECOND PHYSICAL       ╲
         ╱  INFORMATION AND SECOND   ╲      No
        ⟨  WATER METABOLISM INDEX ARE ⟩ ─────────────▶
         ╲       ACQUIRED?           ╱
          ╲────────────────────────╱
                      │ Yes
                      ▼
        ┌──────────────────────────────┐           S22
        │  DETERMINE METABOLIC HEALTH   │
        │          DEGREE               │
        └──────────────────────────────┘
```

FIG.9

FIG.10

| WT | FIRST PHYSICAL INFORMATION | | | | | | | | FIRST ENVIRONMENTAL INFORMATION | | | | |
|---|---|---|---|---|---|---|---|---|---|---|---|---|---|
| | AGE | GENDER | WEIGHT | FFM | PAL | TEE | SES | Athlete | TEMPERATURE | HUMIDITY | LATITUDE | HEIGHT | HDI |
| 3.5 | 21 | 0 | 73.0 | 60.1 | 2.5 | 12.7 | 1 | 1 | 20.5 | 35 | 30 | 500 | 1 |
| 3.2 | 33 | 0 | 65.0 | 55.0 | 1.8 | 10.0 | 3 | 0 | 15.5 | 25.5 | 50 | 1000 | 3 |
| 2.8 | 24 | 1 | 45.5 | 40.0 | 1.5 | 9.0 | 2 | 0 | 30.2 | 50.3 | 40 | 300 | 2 |
| 2.7 | 58 | 1 | 56.7 | 48.9 | 1.2 | 6.5 | 3 | 0 | 8.5 | 24.7 | 60 | 700 | 3 |
| 3.8 | 42 | 0 | 85.0 | 76.3 | 2.5 | 15.4 | 1 | 1 | 24.5 | 34.5 | 45 | 300 | 1 |
| 2.5 | 47 | 1 | 50.3 | 43.0 | 2.1 | 11.2 | 1 | 1 | 15.8 | 30.5 | 50 | 200 | 1 |
| 2.9 | 29 | 1 | 52.8 | 49.7 | 1.4 | 10.4 | 2 | 0 | 35.0 | 33.2 | 10 | 1200 | 2 |
| ⋮ | ⋮ | ⋮ | ⋮ | ⋮ | ⋮ | ⋮ | ⋮ | ⋮ | ⋮ | ⋮ | ⋮ | ⋮ | ⋮ |

FIG.11

WT (L/DAY)

TEMPERATURE

FIG.12

FIG.13

```
         ┌──────────────────────────────┐
         │  WATER METABOLISM INDEX ESTIMATION │
         │          PROCESSING          │
         └──────────────────────────────┘
                       │
                       ▼
```

S31

SECOND ENVIRONMENTAL INFORMATION AND
SECOND ENVIRONMENTAL INFORMATION ARE
ACQUIRED?

No

Yes

S12

INDEX MODEL IS ACQUIRED?

No

Yes

S13

DETERMINE SECOND WATER
METABOLISM INDEX

## FIG.14

## FIG.15

## INTERNATIONAL SEARCH REPORT

International application No.

**PCT/JP2023/011378**

### A. CLASSIFICATION OF SUBJECT MATTER

*G16H 50/50*(2018.01)i
FI:   G16H50/50

According to International Patent Classification (IPC) or to both national classification and IPC

### B. FIELDS SEARCHED

Minimum documentation searched (classification system followed by classification symbols)

G16H50/50

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | 柳慶介,生野壮一郎. Android携帯端末を用いた熱中症予防アプリケーションの開発. 電子情報通信学会技術研究報告. 11 January 2018, vol. 117, no. 390, pp. 89-92, ISSN: 0913-5685, in particular, p 90, column 2, lines 17-27, (YANAGI, Keisuke, IKUNO, Soichiro. Development of warning application of heat stroke by Android operating system. IEICE Technical Report.) p. 90, column 2, lines 17-27 | 1-24 |
| Y | WO 2020/203728 A1 (TANITA CORP.) 08 October 2020 (2020-10-08) paragraphs [0002], [0078], [0080] | 1-24 |
| Y | WO 02/053209 A1 (PHILIPS JAPAN, LTD.) 11 July 2002 (2002-07-11) p. 57, lines 26-29, p. 58, lines 1-8 | 18, 21, 24 |
| A | | 117, 1920, 2223 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 May 2023** | **23 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/011378**

| Patent document cited in search report | Publication date (day/month/year) | Patent family member(s) | Publication date (day/month/year) |
|---|---|---|---|
| WO 2020/203728 A1 | 08 October 2020 | JP 2020-162834 A<br>paragraphs [0001], [0080], [0082] | |
| WO 02/053209 A1 | 11 July 2002 | US 2005/0102165 A1<br>paragraphs [0493]-[0500]<br>EP 1364666 A1<br>JP 4276834 B2 | |

Form PCT/ISA/210 (patent family annex) (January 2015)

**REFERENCES CITED IN THE DESCRIPTION**

**Patent documents cited in the description**

- JP 2022042721 A

- JP 2022132055 A